# EUROPEAN PATENT SPECIFICATION

(11) **EP 2 782 458 B1**
(45) Date of publication and mention of the grant of the patent: **12.09.2018**
(21) Application number: 12787031.9
(22) Date of filing: 19.11.2012
(51) Int. Cl.: A23K 10/18, A23K 20/174, A23K 20/189, A23K 10/14, A23K 20/24, A23K 20/22, A23K 20/26, A23K 20/28, A23K 20/20, A23K 50/70, A23K 50/75, A61K 38/48, A61K 39/012, A23K 20/10, A61K 38/47

(54) **USE OF ACID STABLE PROTEASES IN ANIMAL FEED TO INCREASE PERFORMANCE OF COCCI-VACCINATED BROILER CHICKENS**
VERWENDUNG VON SÄURESTABILEN PROTEASEN IN TIERFUTTER UM DIE LEISTUNGSFÄHIGKEIT VON GEGEN KOKzidien GEIMPFTEN MASTHÜHNERN ZU ERHÖHEN
UTILISATION DE PROTÉASES STABLES EN MILIEU ACIDE DANS DES ALIMENTS POUR ANIMAUX POUR AUGMENTER LES PERFORMANCES DE JEUNES POULETS VACCINÉS CONTRE LES COCCIdies

(30) Priority: 17.11.2011 EP 11189542
(43) Date of publication of application: 01.10.2014
(73) Proprietor: DSM IP Assets B.V., 6411 TE Heerlen (NL); Novozymes A/S, 2880 Bagsværd (DK)
(72) Inventor: WARD , Nelson, Parsippany, NJ 07054 (US); KNAP, Inge, CH-4002 Basel (CH)
(74) Representative: NZ EPO Representatives
(86) International application number: PCT/EP2012/072979
(87) International publication number: WO 2013/072521

(56) References cited:
- WO-A2-01/58276
- WO-A2-2012/110778
- US-A1- 2006 134 091
- US-A1- 2007 104 764
- US-A1- 2012 225 050
- PEEK H W ET AL: "Dietary protease can alleviate negative effects of a coccidiosis infection on production performance in broiler chickens", ANIMAL FEED SCIENCE AND TECHNOLOGY, ELSEVIER, AMSTERDAM, NL, vol. 150, no. 1-2, 30 March 2009 (2009-03-30), pages 151-159, XP026031558, ISSN: 0377-8401, DOI: 10.1016/J.ANIFEEDSCI.2008.08.006 [retrieved on 2008-09-24]
- WILLIAMS R B ET AL: "The efficacy and economic benefits of paracox, a live attenuated anticocidial vaccine, in commercial trials with standard broiler chickens in the United Kingdom", INTERNATIONAL JOURNAL OF PARASITOLOGY, PERGAMON PRESS, GB, vol. 29, 1 February 1999 (1999-02-01), pages 341-355, XP002969285, ISSN: 0020-7519, DOI: 10.1016/S0020-7519(98)00212-4

### SEQUENCE LISTING

<110> DSM IP Assets
<120> Additions of ProAct to cocci-vaccinated birds
<130> 28320
<160> 2
<170> PatentIn version 3.5
<210> 1
   <211> 188
   <212> PRT
   <213> Nocardioides sp. NRRL 18262
<400> 1
   Ala Asp Ile Ile Gly Gly Leu Ala Tyr Thr Met Gly Gly Arg Cys Ser
   1 5 10 15
   Val Gly Phe Ala Ala Thr Asn Ala Ala Gly Gln Pro Gly Phe Val Thr
   20 25 30
   Ala Gly His Cys Gly Arg Val Gly Thr Gln Val Thr Ile Gly Asn Gly
   35 40 45
   Arg Gly Val Phe Glu Gln Ser Val Phe Pro Gly Asn Asp Ala Ala Phe
   50 55 60
   Val Arg Gly Thr Ser Asn Phe Thr Leu Thr Asn Leu Val Ser Arg Tyr
   65 70 75 80
   Asn Thr Gly Gly Tyr Ala Ala Val Ala Gly His Asn Gln Ala Pro Ile
   85 90 95
   Gly Ser Ser Val Cys Arg Ser Gly Ser Thr Thr Gly Trp His Cys Gly
   100 105 110
   Thr Ile Gln Ala Arg Gly Gln Ser Val Ser Tyr Pro Glu Gly Thr Val
   115 120 125
   Thr Asn Met Thr Arg Thr Thr Val Cys Ala Glu Pro Gly Asp Ser Gly
   130 135 140
   Gly Ser Tyr Ile Ser Gly Thr Gln Ala Gln Gly Val Thr Ser Gly Gly
   145 150 155 160
   Ser Gly Asn Cys Arg Thr Gly Gly Thr Thr Phe Tyr Gln Glu Val Thr
   165 170 175
   Pro Met Val Asn Ser Trp Gly Val Arg Leu Arg Thr
   180 185
<210> 2
   <211> 17
   <212> PRT
   <213> Nocardiopsis alba
<400> 2
   Ala Asp Ile Ile Gly Gly Leu Ala Tyr Thr Met Gly Gly Arg Cys Ser
   1               5                   10                  15
   Val

## Description

### Technical Field

The present invention relates to the use of acid-stable proteases in animal feed in combination with anti-coccidial agents, in particular to the use of such proteases for increasing the performance of vaccinated animals.

The use of proteases in animal feed is known from the following documents: W095/28850 discloses an animal feed additive comprising a phytase and a proteolytic enzyme. Various proteolytic enzymes are specified at p. 7.

W096/05739 discloses an enzyme feed additive comprising xylanase and a protease. Suitable proteases are listed at p.25.W095/02044 discloses proteases derived from Aspergillus aculeatus, as well as the use in animal feed thereof.

US 3966971 discloses a process of obtaining protein from a vegetable protein source by treatment with an acid phytase and optionally a proteolytic enzyme. Suitable proteases are specified in column 2.

US 2006/134091 discloses the use of a combination of a protease and an inner salt of a quaternary amine carboxylic acid for the preparation of an agent for the treatment and/or prophylaxis of coccidiosis and bacterial infections such as necrotic enterities in animals.

PEEK H W ET AL: "Dietary protease can alleviate negative effects of a coccidiosis infection on production performance in broiler chickens", ANIMAL FEED SCIENCE AND TECHNOLOGY, ELSEVIER, AMSTERDAM, NL, vol. 150, no. 1-2, 30 March 2009, pages 151-159 also discloses experiments to determine the effect of dietary protease on coccidiosis infection.

J Parker ET AL: "Enzymes as feed additive to aid in responses against Eimeria species in coccidia-vaccinated broilers fed corn-soybean meal diets with different protein levels", Poultry science, 1 April 2007, pages 643-653 and C. L. Walk ET AL: "Effects of dietary enzymes on performance and intestinal goblet cell number of broilers exposed to a live coccidia oocyst vaccine", Poultry science, vol. 90, no. 1, 21 December 2010, pages 91-98 disclose research to evaluate the effects of dietary enzymes on broilers vaccinated a live coccidia oocyst vaccine.

### Technical Background

Coccidia is a generic name given to single cell protozoan organisms that are intestinal parasites that infect both vertebrates and invertebrates. The organisms cause coccidiosis, and usually settle in the small intestine, such as the colon. Infection with coccidia for farm animals can not only seriously reduce growth, but it can be lifethreatening. Symptoms from coccidial infection include loss of epithelial cells, the denuding of gut mucosa, and diarrhoea (often with a concomitant loss of blood). For some farm animals, such as poultry, coccidial infection can be fatal, if not seriously damaging to the animal's health.

Poultry are particularly vulnerable for coccidiosis because of several reasons: (1) The parasitic cycle of 6 to 8 days hits them at a critical stage between weeks 2 and week 4, when maximum growth is usually expressed. Since the parasites virtually destroy the whole intestinal epithelium, the absorption of nutrients is dramatically reduced, which results in marked growth depression. Until slaughter at 5 or 6 weeks, there is not enough time to recover. (2) There are 7 species of Eimeria which can infect poultry, more than in any other animal category, and at least 4 of them are regularly seen in commercial operations. Thus, when one infectious cycle is concluded already another one can be at an early stage so that coccidiosis becomes chronic. (3) In poultry the most pathogenic species (Eimeria tenella, E. necatrix) are observed, which induce severe hemorrhages and in certain cases can cause a mortality of up to 50%. Such an acute case of coccidiosis could easily ruin a poultry farmer. (4) The intensive husbandry of poultry (100,000 chicks or more in one house) on deep litter facilitates access of poultry to the infectious stages of coccidia in the faeces via coprophagy and thus supports a fast spreading of the disease through a whole poultry flock. If the sanitary conditions are not rigorous, the disease will also transfer to other poultry houses on the same farm and stay on site for years.

In order to combat coccidiosis, animal feed is often supplemented with a coccidiostat and/or animals are vaccinated with a drug as for example with Coccivac®B, an anticoccidial vaccine of Shering-Plough Animal Health Corporation. Coccidiostats that have been approved by the EEC for use with poultry (chickens, turkeys, broilers and laying hens) include sulphonimides, amprolium, decoquinate, and ionophores.

It is well known that cocci-vaccination causes decrease of the performance of broiler chicken. In particular, cocci-vaccination causes reduction in feed intake and feed efficiency. Currently 20% of all broiler produced in US are vaccinated against coccidiocis. It is expected that the use of cocci-vacination will increase to 50% by 2020 in the broiler production in US. Therefore the use of cocci-vaccination has an enormous economic impact for the broiler producers.

### Summary of the Invention

It has been found surprisingly that the addition of at least one acid stable protease as defined hereineafter to the diet of cocci-vaccinated birds results in a significant improvement of animal performance. The inventors found that especially the use of serin proteases in combination with cocci-vaccination improves the performance of broiler chicken. In particular the use of serin proteases increases weight gain and improves Feed Conversion Ratio (FCR) of cocci-vaccinated birds. Therefore the use of such proteases can overcome the problems caused by the use of the cocci-vaccination.

The term "birds" includes poultry such as turkeys, ducks and chickens (including but not limited to broiler chicks, layers).

### Detailed Description of the Invention

Proteases are classified on the basis of their catalytic mechanism into the following groups: serine proteases (S), cysteine proteases (C), aspartic proteases (A), metalloproteases (M), and unknown, or as yet unclassified, proteases (U), see Handbook of Proteolytic Enzymes, A. J. Barrett, N. D. Rawlings, J. F. Woessner (eds), Academic Press (1998), in particular the general introduction part.

Proteases for use according to the invention are acid stable proteases. Proteases according to the invention are acid stable serine proteases. The term serine protease refers to serine peptidases and their clans as defined in the above Handbook. In the 1998 version of this handbook, serine peptidases and their clans are dealt with in chapters 1-175. Serine proteases may be defined as peptidases in which the catalytic mechanism depends upon the hydroxyl group of a serine residue acting as the nucleophile that attacks the peptide bond. Examples of serine proteases for use according to the invention are proteases of Clan SA, e. g. Family S2 (Streptogrisin), e. g. Sub-family S2A (alpha-lytic protease), as defined in the above Handbook. Protease activity can be measured using any assay, in which a substrate is employed, that includes peptide bonds relevant for the specificity of the protease in question. Assay-pH and assay-temperature are likewise to be adapted to the protease in question. Examples of assay-pH-values are pH 5, 6, 7, 8, 9, 10, or 11. Examples of assay-temperatures are 30, 35, 37, 40, 45, 50, 55, 60, 65 or 70 °C.

Examples of protease substrates are casein, and pNA-substrates, such as Suc-AAPF-NA (available e. g. from Sigma S7388). The capital letters in this pNA-substrate refers to the one-letter amino acid code. Another example is Protazyme AK (azurine-dyed crosslinked casein prepared as tablets by Megazyme T-PRAK). For pH-activity and pH-stability studies, the pNA-substrate is preferred, whereas for temperature activity studies, the Protazyme AK substrate is preferred.

There are no limitations on the origin of the acid stable serine protease for use according to the invention. Thus, the term protease includes not only natural or wild-type proteases, but also any mutants, variants, fragments etc. thereof exhibiting protease activity, as well as synthetic proteases, such as shuffled proteases, and consensus proteases. Such genetically engineered proteases can be prepared as is generally known in the art, e. g. by Site-directed Mutagenesis, by PCR (using a PCR fragment containing the desired mutation as one of the primers in the PCR reactions), or by Random Mutagenesis. The preparation of consensus proteins is described in e. g. EP 0 897 985.

Examples of acid-stable proteases used are
a) the proteases derived from Nocardiopsis sp. NRRL 18262, and Nocardiopsis alba ;
b) proteases of at least 60, 65, 70, 75, 80, 85, 90, or at least 95% amino acid identity to any of the proteases of (i) ;
c) proteases of at least 60, 65, 70, 75, 80, 85, 90, or at least 95% identity to any of SEQ ID NO : 1, and/or SEQ ID NO : 2.

For calculating percentage identity, any computer program known in the art can be used. Examples of such computer programs are the Clustal V algorithm (Higgins, D. G., and Sharp, P. M. (1989), Gene (Amsterdam), 73, 237-244 ; and the GAP program provided in the GCG version 8 program package (Program Manual for the Wisconsin Package, Version 8, Genetics Computer Group, 575 Science Drive, Madison, Wisconsin, USA 53711) (Needleman, S. B. and Wunsch, C. D., (1970), Journal of Molecular Biology, 48, 443-453.

In a particular embodiment, the protease used is a microbial protease, the term microbial indicating that the protease is derived from, or originates from a microorganism, or is an analogue, a fragment, a variant, a mutant, or a synthetic protease derived from a microorganism. It may be produced or expressed in the original wild-type microbial strain, in another microbial strain, or in a plant; i. e. the term covers the expression of wild-type, naturally occurring proteases, as well as expression in any host of recombinant, genetically engineered or synthetic proteases. Examples of microorganisms are bacteria, e. g. bacteria of the phylum Actinobacteria phy. nov., e. g. of class I: Actinobacteria, e. g. of the Subclass V: Actinobacteridae, e. g. of the Order I: Actinomycetales, e. g. of the Suborder XII: Streptosporangineae, e. g. of the Family II: Nocardiopsaceae, e. g. of the Genus I: Nocardiopsis, e. g. Nocardiopsis sp. NRRL 18262, and Nocardiopsis alba ; e.g. of the species Bacillus or mutants or variants thereof exhibiting protease activity. This taxonomy is on the basis of Berge's Manual of Systematic Bacteriology, 2nd edition, 2000, Springer (preprint: Road Map to Bergey's).

Further examples of microorganisms are fungi, such as yeast or filamentous fungi. In the use according to the invention the protease can be fed to the animal before, after, or simultaneously with the diet of the cocci-vaccinated bird. The latter is preferred.

In the present context, the term acid-stable means, that the protease activity of the pure protease enzyme, in a dilution corresponding to A₂₈₀ = 1.0, and following incubation for 2 hours at 37 C in the following buffer:
- 100mM succinic acid, 100mM HEPES, 100mM CHES,
- 100mM CABS, 1mM CaCl₂, 150mM KCI, 0.01 % Triton®X-100, pH 3.5, is at least 40% of the reference activity, as measured using the assay described in Example 1 herein (substrate : Suc-AAPF-pNA, pH 9. 0, 25°C).

In particular embodiments of the above acid-stability definition, the protease activity is at least 45, 50, 55, 60, 65, 70, 75, 80, 85, 90, 95, or at least 97% of the reference activity.

The term reference activity refers to the protease activity of the same protease, following incubation in pure form, in a dilution corresponding to A₂₈₀ = 1.0, for 2 hours at 5 C in the following buffer: 100mM succinic acid, 100mM HEPES, 100mM CHES, 100mM CABS, 1mM CaCl₂, 150mM KCI, 0.01 % Triton®X-100, pH 9.0, wherein the activity is determined as described above.

In other words, the method of determining acid-stability comprises the following steps:
a) The protease sample to be tested (in pure form, A₂₈₀ = 1.0) is divided in two aliquots (I and II);
b) Aliquot I is incubated for 2 hours at 37°C and pH 3.5;
c) Residual activity of aliquot I is measured (pH 9.0 and 25°C);
d) Aliquot II is incubated for 2 hours at 5°C and pH 9.0;
e) Residual activity of aliquot II is measured (pH 9.0 and 25°C);
f) Percentage residual activity of aliquot I relative to residual activity of aliquot II is calculated.

Alternatively, in the above definition of acid stability, the step b) buffer pH-value may be 1.0, 1.5, 2.0, 2.5, 3.0, 3.1, 3.2, 3.3, or 3.4.

In other alternative embodiments of the above acid stability definition relating to the above alternative step b) buffer pH-values, the residual protease activity as compared to the reference, is at least 5, 10, 15, 20, 25, 30, 35, 40, 45, 50, 55, 60, 65, 70, 75, 80, 85, 90, 95, or at least 97%.

In alternative embodiments, pH values of 6.0, 6.5, 7.0, 7.5, 8.0, or 8.5 can be applied for the step d) buffer.

In the above acid-stability definition, the term A₂₈₀ = 1.0 means such concentration (dilution) of said pure protease which gives rise to an absorption of 1.0 at 280 nm in a 1 cm path length cuvette relative to a buffer blank.

And in the above acid-stability definition, the term pure protease refers to a sample with a A₂₈₀/A₂₆₀ ratio above or equal to 1.70.

In another particular embodiment, the protease for use according to the invention, besides being acid-stable, is also thermostable.

The term thermostable means one or more of the following: That the temperature optimum is at least 50 °C, 52 °C, 54 °C, 56 °C, 58 °C, 60 °C, 62 °C, 64 °C, 66 °C, °68 C, or at least °70 C.

The protease should of course be applied in an effective amount, i. e. in an amount adequate for improving the feed conversion ratio in birds vaccinated with an anti-coccidial vaccine.

In a preferred example, the intended dosage of the protease is 0.01-200 mg protease enzyme protein per kg final feed.

The term feed conversion ratio is determined on the basis of a growth trial comprising a first treatment in which the composition according to the invention is added to the animal feed in a suitable concentration per kg feed, and a second treatment (control) with no addition of the composition to the animal feed.

As it is generally known, an improved FCR is lower than the control FCR. In particular embodiments, the FCR is improved (i.e. reduced) as compared to the control by at least 1.0% or 5%.

For the uses according to the invention, the protease need not be that pure; it may e. g. include other enzymes, even other acid stable proteases, in which case it could be termed am enzyme or protease preparation. Nevertheless, a well-defined enzyme / protease preparation is advantageous. For instance, it is much easier to dose correctly to the feed a protease that is essentially free from interfering or contaminating other proteases. The term dose correctly refers in particular to the objective of obtaining consistent and constant results, and the capability of optimising dosage based upon the desired effect.

In a preferred embodiment of a poultry feeding concept, the protease is used in form of a feed additive.

The incorporation of the composition of feed additives as exemplified herein above to poultry feeds is in practice carried out using a concentrate or a premix. A premix designates a preferably uniform mixture of one or more micro-ingredients with diluent and/or carrier. Premixes are used to facilitate uniform dispersion of micro-ingredients in a larger mix. A premix according to the invention can be added to feed ingredients or to the drinking water as solids (for example as water soluble powder) or liquids.

A part from the acid stable protease of the invention, animal feed additives of the invention contain at least one fat-soluble vitamin, and/or at least one water soluble vitamin, and/or at least one trace mineral, and/or at least one macro mineral.

Further, optional, feed-additive ingredients are coloring agents, e.g. carotenoids such as beta-carotene, astaxanthin, canthaxanthin, apoester and lutein; aroma compounds; stabilisers; antimicrobial peptides; polyunsaturated fatty acids (PUFAs); reactive oxygen generating species; and/or at least one enzyme selected from amongst phytase (EC 3.1.3.8 or 3.1.3.26); xylanase (EC 3.2.1.8); galactanase (EC 3.2.1.89); alpha-galactosidase (EC 3.2.1.22); protease (EC 3.4., phospholipase A1 (EC 3.1.1.32); phospholipase A2 (EC 3.1.1.4); lysophospholipase (EC 3.1.1.5); phospholipase C (EC 3.1.4.3); phospholipase D (EC 3.1.4.4); amylase such as, for example, alpha-amylase (EC 3.2.1.1); and/or beta-glucanase (EC 3.2.1.4 or EC 3.2.1.6).

Examples of antimicrobial peptides (AMP's) are CAP18, Leucocin A, Protegrin-1, Thanatin, Defensin, Lactoferrin, Lactoferricin, and Ovispirin such as Novispirin (Robert Lehrer, 2000), Plectasins, and Statins.

Examples of polyunsaturated fatty acids are C18, C20 and C22 polyunsaturated fatty acids, such as arachidonic acid, docosohexaenoic acid, eicosapentaenoic acid and gamma-linoleic acid.

Examples of reactive oxygen generating species are chemicals such as perborate, persulphate, or percarbonate; and enzymes such as an oxidase, an oxygenase or a syntethase.

Usually fat- and water-soluble vitamins, as well as trace minerals form part of a so-called premix intended for addition to the feed, whereas macro minerals are usually separately added to the feed.

The following are non-exclusive lists of examples of these components:
- Examples of fat-soluble vitamins are vitamin A, vitamin D3, vitamin E, and vitamin K, e.g. vitamin K3.
- Examples of water-soluble vitamins are vitamin B12, biotin and choline, vitamin B1, vitamin B2, vitamin B6, niacin, folic acid and panthothenate, e.g. Ca-D-panthothenate.
- Examples of trace minerals are manganese, zinc, iron, copper, iodine, selenium, and cobalt.
- Examples of macro minerals are calcium, phosphorus and sodium.

A premix can contain, for example, per ton of poultry feed, 50 to 200 g of a propylene glycol solution of the mixture of the active compounds, 20 to 1000 g of an emulsifying agent, 50 to 900 g of cereals and by-products, 20 to 100 g of a proteinic support (milk powder, casein, etc) and 50 to 300 g of a mineral component (expanded silica, feed quality lime, bi-calcium phosphate, etc).

A feed additive or premix as described above is finally added the animal feed composition. It is prepared and added such that the amount of the protease corresponds to an intended addition of 0.01 - 200 mg protease protein per kg feed.

Animal feed compositions or diets have a relatively high content of protein. According to the National Research Council (NRC) publications referred to above, poultry and pig diets can be characterised as indicated in Table B of
WO 01/58276. An animal feed composition according to the invention has a crude protein content of 50-800 g/kg, and furthermore comprises at least one protease as claimed herein.

Furthermore, or in the alternative (to the crude protein content indicated above), the animal feed composition of the invention has a content of metabolisable energy of 10 - 30 MJ/kg; and/or a content of calcium of 0.1 - 200 g/kg; and/or a content of available phosphorus of 0.1 - 200 g/kg; and/or a content of methionine of 0.1 - 100 g/kg; and/or a content of methionine plus cysteine of 0.1 - 150 g/kg; and/or a content of lysine of 0.5-50 g/kg.

In particular embodiments, the content of metabolisable energy, crude protein, calcium, phosphorus, methionine, methionine plus cysteine, and/or lysine is within any one of ranges 2, 3, 4 or 5 as disclosed in Table B of WO 01/58276.

For determining mg protease protein per kg feed, the protease is purified from the feed composition, and the specific activity of the purified protease is determined using a relevant assay (see under protease activity, substrates, and assays). The protease activity of the feed composition as such is also determined using the same assay, and on the basis of these two determinations, the dosage in mg protease protein per kg feed is calculated.

The same principles apply for determining mg protease protein in feed additives.

The protease of Nocardiopsis sp. NRRL 18262 according to the invention can be prepared using conventional methods, as generally described in WO01/58276. A feed additive comprising the protease of Nocardiopsis sp. NRRL 18262 is also commercially available (for example as Ronozyme®ProAct, supplied by DSM Nutritional Products, Kaiseraugst, Switzerland) or can easily be prepared by a skilled person using processes and methods well-known in the prior art.

The invention is according to the appended claims.

The following examples further illustrate the invention.

### Examples

### Example 1 pH-stability assay

Suc-AAPF-pNA (Sigma S-7388) was used for obtaining pH stability profiles.

Assay buffer: 100 mM succinic acid, 100 mM HEPES, 100 mM CHES, 100 mM CABS, 1mM CaCl₂, 150mM KCl, 0.01% Triton®X-100 adjusted to pH-values 2.0, 2.5, 3.0, 3.5, 4.0, 4.5, 5.0, 6 0, 7.0, 8.0, 9.0, 10.0 or 11.0 with HCl or NaOH.

Each protease sample (in 1mM succinic acid, 2mM CaCl₂, 100mM NaCl, pH 6.0 and with an A₂₈₀ absorption > 10) was diluted in the assay buffer at each pH value tested to A₂₈₀ = 1.0. The diluted protease samples were incubated for 2 hours at 37°C.

After incubation, protease samples were diluted in 100 mM succinic acid, 100 mM HEPES, 100 mM CHES, 100 mM CABS, 1 mM CaCl₂, 150 mM KCl, 0.01% Triton®X-100, pH 9.0, bringing the pH of all samples to pH 9.0.

In the following activity measurement, the temperature was 25°C.

300µl diluted protease sample was mixed with 1.5ml of the pH 9.0 assay buffer and the activity reaction was started by adding 1.5ml pNA substrate (50mg dissolved in 1.0 ml DMSO and further diluted 45x with 0.01% Triton®X-100) and, after mixing, the increase in A₄₀₅ was monitored by a spectrophotometer as a measurement of the (residual) protease activity.

The 37 C incubation was performed at the different pH-values and the activity measurements were plotted as residual activities against pH. The residual activities were normalized with the activity of a parallel incubation (control), where the protease was diluted to A₂₈₀ = 1.0 in the assay buffer at pH 9.0 and incubated for 2 hours at 5°C before activity measurement as the other incubations. The protease samples were diluted prior to the activity measurement in order to ensure that all activity measurements fell within the linear part of the dose-response curve for the assay.

### Example 2 - Feeding of vaccinated birds with acid stable proteases

A floor pen trial was conducted using day old male birds Ross 708. A total of 27 pens were used in the trial and 10 birds were placed per pen on day 1 of age. The pens were randomly allocated across 3 treatments with 9 replicates/ treatment. The birds were fed nutritionally-adequate basal diets based on corn/soybean meal and adjusted to the bird age by feeding a starter diet from day 1 to 21 and a grower diet from day 22 to 42.

### Treatment 1: Basal Diet

Cocci Vac® B on day 1 of age + dietary Monteban® (coccidiostat) 80 ppm. All birds received Cocci Vac® B on day 1 of age administered nasally in the hatchery.

### Treatment 2:

Cocci Vac® B on day 1 of age without Monteban®

### Treatment 3:

as Treatment 2 plus 400 ppm RONOZYME® enzyme blend (supplied by DSM Nutritional Products, Kaiseraugst, Switzerland)

Monteban was fed to suppress the effect of the Cocci Vac. Cocci-Vac induces immunity to coccidiosis by causing a low level of the disease.

The enzyme blend comprised:
- 200 ppm RONOZYME®ProAct (acid stable protease) and
- 100 ppm RONOZYME WX + 50 ppm RONOZYME A + 50 ppm ROXAZYME G2 (= enzyme mixture containing xylanase, amylase, cellulose, beta-glucanase and others)

At the end of the trial at day 42 the average bird weight and the average feed consumption were determined and the Feed Conversion Ration (FCR) was calculated by dividing the feed intake by the weight gain for each treatment.

The results are summarized in the following tables, wherein table 1 lists body weight at day 42 and table 2 lists the FCR at day 42.

**Table 1. Average 42-day Body Weight**

| | **Average body weight g** |
|---|---|
| **Treatment 1** | 2832^{a} |
| **Treatment 2** | 2546^{c} |
| **Treatment 3** | 2710^{b} |

| | |
|---|---|
| ^{abc}P<0.05 | |

**Table 2. Feed Conversion Ration day 42**

| | **FCR** |
|---|---|
| **Treatment 1** | 1.544^{b} |
| **Treatment 2** | 1.656^{a} |
| **Treatment 3** | 1.559^{b} |

| | |
|---|---|
| ^{abc}P<0.05 | |

The results clearly show that cocci vaccination (Treatment 2) decreased body weights and adversely affected FCR, whereas the coccidiostat (Treatment 1) eliminated these effects. The addition of the enzyme blend resulted in significant higher body weight and improved FCR compared to Treatment 2. Addition of the enzyme blend to the birds that experienced coccidiosis brought the performance of the birds to that near of birds that were fed the coccidiostat.

### Example 3 - Feeding of vaccinated birds with acid stable proteases

A floor pen trial was conducted using day old male birds Cobb X Cobb chicks. A total of 2,160 were allocated to the study. The experiment consisted of 48 pens of 45 male broiler chickens. Treatments were replicated in eight (8) blocks with six (6) treatments randomized in each. The birds were fed nutritionally-adequate basal diets based on corn/soybean meal and adjusted to the bird age by feeding a starter diet from day 1 to 16 and a grower diet from day 17 to 32 and a finisher diet from day 33 to 42. The 6 treatment were as follow:

### Treatments

| Treatment | 1-16 | 17-32 | 33-42 |
|---|---|---|---|
| 1. PC (Salinomycin) | -- | -- | -- |
| 2. NC (Cocci Vac® B) | -- | -- | -- |
| 3. NC (Cocci Vac® B) | 200 ppm ProAct | 200 ppm ProAct | 200 ppm ProAct |
| 4. NC (Cocci Vac® B) | 200 ppm ProAct + 200 ppm Rono AX | 200 ppm ProAct + 200 ppm Rono AX | 200 ppm ProAct + 200 ppm Rono AX |
| 5. NC (Cocci Vac® B) | 200 ppm ProAct + 160 ppm WX | 200 ppm ProAct + 160 ppm WX | 200 ppm ProAct + 160 ppm WX |
| 6. NC (Cocci Vac® B) | 200 ppm ProAct + 100 ppm Roxazyme | 200 ppm ProAct + 100 ppm Roxazyme | 200 ppm ProAct + 100 ppm Roxazyme |

| | | | |
|---|---|---|---|
| As treatment RONOZYME enzyme products, supplied by DSM Nutritional Products, Kaiseraugst, Switzerland have been used. Ronozyme® AX = 80% Ronozyme® WX + 20% Ronozyme® A Ronozyme® ProAct = pure protease product. | | | |

All birds received Cocci Vac® B on day 1 of age administered nasally in the hatchery except the positive control birds that were given a standard dose of the coccidiostat Salinomcyin of 60 g/ tone of feed throughout the study

Salinomcyin is the most used coccidiostate that suppress development of the very common decease coccidiosis.

Cocci Vac® B induces immunity to coccidiosis by causing a low level of the disease.

At the end of the trial at day 42 the average bird weight (Avg. Wt. Gain) and the average feed consumption (Feed Consump.) were determined and the Feed Conversion Ration (FRC) was calculated by dividing the feed intake by the weight gain for each treatment

| | | **Day 42** | |
|---|---|---|---|
| | **Feed** | **FCR** | **Avg. Wt.** |
| **Treatment** | **Consump.** | | **Gain** |
| 1. Salinomcyin 60 g/t | 191.58 a | 1.719 d | 2.490 a |
| 2. Cocci Vac® B (CVB) | 189.96 ab | 1.792 a | 2.372b |
| 3. CVB ProAct 200 ppm | 187.85 bc | 1.760 b | 2.412 b |
| 4. CVB ProAct 200 ppm+ Rono AX 200 ppm | 184.91 c | 1.724 cd | 2.400 b |
| 5. CVB ProAct 200 ppm+ Rono WX 200 ppm | 181.27 d | 1.747 bc | 2.393 b |
| 6. CVB ProAct 200 ppm+Roxazyme 100 ppm | 186.16 c | 1.712 d | 2.472 a |

Cocci Vac (Treatment 2) decreased body weights and adversely affected FCR, compared to the PC given the Salinomcyin treatment.

The addition of the protease RONOZYME ProAct on top of the Cocci Vac® B resulted in significant improved FCR compared to the Cocci Vac (Treatment 2). Addition of any of the enzyme blend together with the RONOZYME PROACT product resulted in even further improvement of the to the birds that experienced coccidiosis brought the performance of the birds to that near of birds that were fed the coccidiostat.

## Claims

1. Use of at least one acid-stable protease in combination with anti-coccidial vaccination of birds for increasing weight gain and/or improving the Feed Conversion Ratio (FCR) in the animal compared to a second treatment (control) with no addition of the protease to the animal feed, wherein the protease has an identity of at least 70% to SEQ ID NO: 1.

2. Use according to claim 1, wherein the protease is used in feed for birds which includes poultry such as turkeys, ducks and chickens.

3. The use of claim 2, wherein the intended dosage of the protease is 0.01 - 200 mg protease enzyme protein per kg feed.

4. The use according to any of claims 1 to 3, wherein the FCR is improved by at least 1.0% compared to the control.

5. The use according to claim 4, wherein the FCR is improved by at least 5.0% compared to the control.

6. The use according to any of claims 1 to 5, wherein the use further comprises a phytase, xylanase, galactanase, beta-glucanase, amylase and/or cellulose.

## Patentansprüche

1. Verwendung von mindestens einer säurestabilen Protease in Kombination mit Antikokzidien-Impfung von Vögeln zum Erhöhen von Gewichtszunahme und/oder dem Verbessern der Futterumwandlungsrate (FCR) im Tier verglichen zu einer zweiten Behandlung (Kontrolle) ohne Zugabe der Protease zum Tierfutter, wobei die Protease eine Identität von mindestens 70% zu SEQ ID NO: 1 aufweist.

2. Verwendung nach Anspruch 1, wobei die Protease in Futter für Vögel verwendet wird, was Geflügel wie Truthühner, Enten und Hühner einschließt.

3. Verwendung nach Anspruch 2, wobei die beabsichtigte Dosierung der Protease 0,01 - 200 mg Protease-Enzymprotein pro kg Futter beträgt.

4. Verwendung nach einem beliebigen der Ansprüche 1 bis 3, wobei die FCR um mindestens 1,0% verglichen zur Kontrolle verbessert wird.

5. Verwendung nach Anspruch 4, wobei die FCR um mindestens 5,0% verglichen zur Kontrolle verbessert wird.

6. Verwendung nach einem beliebigen der Ansprüche 1 bis 5, wobei die Verwendung des Weiteren eine Phytase, Xylanase, Galactanase, beta-Glucanase, Amylase und/oder Cellulose umfasst.

## Revendications

1. Utilisation d'au moins une protéase stable en milieu acide en combinaison avec une vaccination anticoccidienne d'oiseaux pour augmenter le gain de poids et/ou améliorer l'indice de consommation (IC) chez l'animal comparativement à un second traitement (témoin) sans aucune addition de la protéase à l'aliment pour animaux, dans laquelle la protéase présente une identité d'au moins 70 % avec SEQ ID NO : 1.

2. Utilisation selon la revendication 1, dans laquelle la protéase est utilisée dans des aliments pour oiseaux qui comprennent la volaille telle que les dindes, les canards et les poulets.

3. Utilisation selon la revendication 2, dans laquelle le dosage prévu de la protéase est de 0,01 à 200 mg de protéine enzyme protéase par kg d'aliment.

4. Utilisation selon l'une quelconque des revendications 1 à 3, dans laquelle l'IC est amélioré d'au moins 1,0 % comparativement au témoin.

5. Utilisation selon la revendication 4, dans laquelle l'IC est amélioré d'au moins 5,0 % comparativement au témoin.

6. Utilisation selon l'une quelconque des revendications 1 à 5, dans laquelle l'utilisation comprend en outre une phytase, une xylanase, une galactanase, une bêta-glucanase, une amylase et/ou de la cellulose.
